# EUROPEAN PATENT SPECIFICATION

(11) **EP 2 837 380 B1**
(45) Date of publication and mention of the grant of the patent: **03.04.2019**
(21) Application number: 13775164.0
(22) Date of filing: 25.02.2013
(51) Int. Cl.: A61K 9/32, A61K 31/4178, A61K 31/4422, A61P 9/12

(54) **LERCANIDIPINE HYDROCHLORIDE AND LOSARTAN POTASSIUM COMPOUND PREPARATION AND PREPARATION METHOD THEREOF**
LERCANIDIPINHYDROCHLORID- UND KALIUMLOSARTAN-KOMBINATIONSPRÄPARAT SOWIE HERSTELLUNGSVERFAHREN DAFÜR
PRÉPARATION CONSTITUÉE DE CHLORHYDRATE DE LERCANIDIPINE ET DE LOSARTAN POTASSIQUE ET SON PROCÉDÉ DE PRÉPARATION

(30) Priority: 11.04.2012 CN 201210104974
(43) Date of publication of application: 18.02.2015
(73) Proprietor: Zhaoke Pharmaceutical (Hefei) Company Limited, Anhui 230088 (CN)
(72) Inventor: LI, Xiaoyi, Hong Kong (CN); ZHANG, Guohui, Hefei Anhui 230088 (CN); DAI, Xiangrong, Hefei Anhui 230088 (CN); LING, Juan, Hefei Anhui 230088 (CN); WU, Yan, Hefei Anhui 230088 (CN)
(74) Representative: dompatent von Kreisler Selting Werner - Partnerschaft von Patent- und Rechtsanwälten mbB
(86) International application number: PCT/CN2013/071838
(87) International publication number: WO 2013/152641

(56) References cited:
- WO-A1-2010/085027
- CN-A- 101 528 203
- CN-A- 102 600 146
- TW-A- 200 501 953
- US-A1- 2004 198 789
- US-A1- 2006 177 507

## Description

### TECHNICAL FIELD OF THE INVENTION

The present invention belongs to the field of medicine, particularly relates to a lercanidipine hydrochloride and losartan potassium compound preparation.

### BACKGROUND OF THE INVENTION

Hypertension is definitely the most important risk factor of cardiovascular disease, the long-term development of which could cause serious lesions to target organs, such as heart, brain, kidney and the like, and the complications of which, such as coronary heart disease, apoplexy, kidney function failure and the like, have high disability rate and lethality. In China, hypertension now has been the first main cause of death. Data indicated that the patients suffering from hypertension have reached 200 million until 2006, while around 1 billion people suffered from hypertension all over the world. Therefore, the prophylaxis and treatment of hypertension, and medication are the most important in the medical research and development task.

Lercanidipine hydrochloride is a third-generation dihydropyridine calcium channel antagonist developed by Recordati corporation, Italy, which was firstly marketed in Netherlands in 1997, subsequently in 18 countries separately, such as France, Australia, Germany and others. The action mechanism of lercanidipine hydrochloride is similar to that of the same kinds of drugs, i.e. reversibly blocking Ca2+ influx of L-type calcium channel in vascular smooth muscle cell membrane, and dilating peripheral vessels to reduce blood pressure. This product has high lipophilicity, thus its onset time is slower while the duration of action is longer. The in vivo/in vitro trials indicate that the negative inotropic action caused by selective exangia of lercanidipine hydrochloride is weaker than that of nifedipine, nitrendipine and felodipine; while its vascular selectivity is higher than those of amlodipine, felodipine, nitrendipine and lacidipine. In addition, lercanidipine hydrochloride also has anti-atherosclerotic and end-organ protective effect. Lercanidipine hydrochloride at therapeutic dose does not interfere with normal cardiac excitability and conductivity of the patients suffering from hypertension. Animal experiments indicate that lercanidipine hydrochloride protect kidney, whose mechanism may be not related to hemodynamics. Lercanidipine hydrochloride has higher vascular selectivity compared with the drugs of the same kind, and its particular lipophilicity makes its blood pressure decreasing effect slow and persistent. Lercanidipine hydrochloride has high safety and no cardiotonic effect, and it does not affect heart rate and has excellent anti-atherosclerotic effect meanwhile, in particular being suitable for hypertension patients accompanying with atherosclerosis, which has high clinical application value and broad market prospect.

Losartan is an oral non-peptide angiotensin II receptor antagonist (AIIA), which was firstly marketed in 1994 and has been approved in 93 countries currently for treating hypertension. Renin-angiotensin system (RAS) in body is activated by renin released by kidney. Renin is capable of decomposing angiotensinogen, a protein derived from liver, and the product after decomposition is angiotensin I. Angiotensin I itself has no bioactivity, however, it is the precursor of Angiotensin II, which has a series of bioactivities on vessels, heart and other tissues in body. AIIA drug losartan is capable of blocking angiotensin receptor (AT1) to avoid angiotensin II binding to the receptor, finally achieving the purpose of preventing vasoconstriction. The clinical application shows that it has better tolerance and fewer side effects.

US 2004/198789 A1 discloses tablets comprising lercanidipine HCl and losartan potassium for treating hypertension.

### SUMMARY OF THE INVENTION

A purpose of the present invention is to provide a lercanidipine and losartan compound preparation, which contains 0.5-40% lercanidipine hydrochloride and 6.25-50% losartan potassium based on mass percentage of the compound preparation, and wherein the mass ratio of lercanidipine hydrochloride to losartan potassium is 30:46.

In the compound preparation provided by the invention, calcium ion antagonist lercanidipine hydrochloride causes reflex sympathetic excitation by dilating vessels, and angiotensin II receptor antagonist losartan could relieve the over-activation of sympathetic system during calcium channel blocker (CCB) decreasing blood pressure by antagonizing neurohormone activity, so as to decrease the adverse effects caused by CCB treatment, which provides a more comprehensive protection to the target organs, such as heart, brain, kidney, etc., thus has a better anti-atherosclerotic effect and therapy of hypertension.

A lercanidipine and losartan compound preparation is specifically disclosed in the embodiments of the invention, the excipients used in which are 20-40% lactose monohydrate, 10-40% microcrystalline cellulose, 10-20% A-type sodium starch glycolate, 2-8% povidone K30, 0.5-2.5% magnesium stearate, 5-15% pregelatinized starch and 1-10% colloidal silicon dioxide.

Preferably, a coating used is opadry white.

The method for preparing said compound preparation includes the following steps:
Step 1: sieved active pharmaceutical ingredients lercanidipine hydrochloride and losartan are stirred and homogenized with lactose monohydrate, microcrystalline cellulose, A-type sodium starch glycolate, povidone K30, magnesium stearate, pregelatinized starch and colloidal silicon dioxide, followed by vacuum compression and milling, sieved and granulated, tabletted with controlling the hardness of tablet core;
Step 2: opadry is added to ethanol with stirring until dispersion, then purified water is added and stirred to obtain isolation coating solution, which is then used for film coating of the tablet core obtained in Step 1;
Step 3: opadry is added to ethanol, stirred and homogenized to obtain enteric coating solution, which is then used to coat enteric coating for the film-coated tablets.

Preferably, the controlled hardness of tablet core in step 1 is 2-10 kg.

Preferably, the concentration of ethanol in step 2 and step 3 is 75-100%.

Preferably, the duration for stirring after adding purified water in step 2 and for the stirring in step 3 is 20-100 min.

Preferably, the opadry in step 2 is Y-1-7000 and the opadry in step 3 is type OY-P, 91S.

The present invention provides a compound preparation, which comprises lercanidipine hydrochloride and losartan potassium as active pharmaceutical ingredients, lactose monohydrate, microcrystalline cellulose, sodium starch glycolate (A-type), povidone K30, magnesium stearate, pregelatinized starch and colloidal silicon dioxide as excipients and opadry white as a coating to formulate the tablet.

In the lercanidipine and losartan compound preparation provided by the present invention, the calcium ion antagonist lercanidipine causes reflex sympathetic excitation by dilating vessels, and after combining with angiotensin II receptor antagonist lorsartan, the anti-neurohormone activity of lorsartan could relieve the over-activation of sympathetic system during calcium channel blocker (CCB) decreasing blood pressure, so as to reduce adverse reactions caused by CCB treatment, which provides a more comprehensive protection to the target organs including heart, brain, kidney, etc.

The clinical trials show that compared with single-ingredient preparation group, the lercanidipine and losartan compound preparation group markedly increases the effectiveness of treatment of mild and moderate hypertension, with markedly reduced incidence of adverse reactions, is tolerable well by patients, and increases the therapy of reducing blood pressure and has excellent clinical application prospects.

### DETAILED DESCRIPTION OF THE INVENTION

The present invention discloses a lercanidipine hydrochloride and losartan potassium compound preparation which could be achieved by those skilled in the art by referring to the contents of the present disclosure. The products, methods and applications of the present disclosure have been described through preferred examples.

In order that the technical solutions of the present invention can be better understood by those skilled in the art, the present invention will be further illustrated in detail by combining with specific examples.

### Example 1: Preparation of a lercanidipine and losartan compound preparation not according to the present invention

Active pharmaceutical ingredients lercanidipine hydrochloride and losartan were weighed and sieved; lercanidipine hydrochloride was 0.5-40% and losartan potassium was 6.25-50%.

Lactose monohydrate, microcrystalline cellulose, sodium starch glycolate (A-type), povidone K30, magnesium stearate, pregelatinized starch and colloidal silicon dioxide were weighed, stirred and homogenized. The excipients comprise 20-40% lactose monohydrate, 10-40% microcrystalline cellulose, 10-20% sodium starch glycolate (A-type), 2-8% povidone K30, 0.5-2.5% magnesium stearate, 5-15% pregelatinized starch and 1-10% colloidal silicon dioxide.

Compressed in vacuum and milled, sieved and granulated; tabletted with controlling the hardness of tablet core.

Formulating isolation coating solution: opadry was added to ethanol with stirring until dispersion, then purified water was added and stirred to formulate isolation coating solution.

Film coating: the prepared isolation coating solution was used to coat film coating for the obtained tablet core to obtain the film-coated tablets.

Formulating enteric coating solution: opadry in prescribed dose was added to ethanol with stirring, and stirring was continued to obtain enteric coating solution.

Enteric coating: the prepared enteric coating solution was used to coat enteric coating for the film-coated tablets, and the unit dose of the obtained compound preparation contained 5 mg lercanidipine and 20 mg losartan.

### Example 2: Preparation of a lercanidipine and losartan compound preparation not according to the present invention

Active pharmaceutical ingredients lercanidipine hydrochloride and losartan were weighed and sieved; lercanidipine hydrochloride was 0.5-40% and losartan potassium was 6.25-50%.

Lactose monohydrate, microcrystalline cellulose, sodium starch glycolate (A-type), povidone K30, magnesium stearate, pregelatinized starch and colloidal silicon dioxide were weighed, stirred and homogenized. The excipients comprise 20-40% lactose monohydrate, 10-40% microcrystalline cellulose, 10-20% sodium starch glycolate (A-type), 2-8% povidone K30, 0.5-2.5% magnesium stearate, 5-15% pregelatinized starch and 1-10% colloidal silicon dioxide.

Compressed in vacuum and milled, sieved and granulated; tabletted with controlling the hardness of tablet core.

Formulating isolation coating solution: opadry was added to ethanol with stirring until dispersion, then purified water was added and stirred to formulate isolation coating solution.

Film coating: the prepared isolation coating solution was used to coat film coating for the obtained tablet core to obtain the film-coated tablets.

Formulating enteric coating solution: opadry in prescribed dose was added to ethanol with stirring, and stirring was continued to obtain enteric coating solution.

Enteric coating: the prepared enteric coating solution was used to coat enteric coating for the film-coated tablets, and the unit dose of the obtained compound preparation contained 7.5 mg lercanidipine and 20 mg losartan.

### Example 3: Assay of lercanidipine hydrochloride in the quality study of a lercanidipine and losartan compound preparation not according to the invention

HPLC method was used to determine the content of lercanidipine hydrochloride in the lercanidipine and losartan compound preparation prepared in Example 1. The detection conditions for liquid chromatography are as shown in table 1.

**Table 1 HPLC detection conditions for lercanidipine hydrochloride in the compound preparation**

| | |
|---|---|
| mobile phase | A: buffer : B: acetonitrile = 50:50 |
| column | C18, Waters 250 mm×4.6mm; 5 µm |
| flow rate | 1.0 ml/min |
| detecting wavelength | 210 nm |
| column temperature | 50°C |
| loading capacity | 20 µl |
| running time | 120 min |

Preparation method of A buffer: 1.36 g monopotassium phosphate was dissolved in 1 L water, after dissolving, 1g octane sulphonic acid sodium salt was added thereto, sonicated and 10% phosphoric acid (v/v) was used to adjust pH to 2.5±0.05.

### Example 4: Pharmacodynamic study on the compound preparation

### Material and method

### 1.1 Material

60 female clean grade spontaneous hypertension rat (SHR) model rats were used, which were around 200 g and the awake tail artery systolic blood pressure (SBP) was 160 mmHg (1 mmHg=0.133 kPa).

### 1.2 Experimental method (the preparations 1 and 3 are not according to the invention)

### 1.2.1 Preparation of experimental model and grouping

The rats were anaesthetized with 50 mg/kg Ketamine Hydrochloride and 5 mg/kg diazepam via intraperitoneal injection, an about 2 cm long skin incision at left groin was cut, arteriae femoralis sheath was exposed, the arteriae femoralis and femoral veins were separated carefully and arteriae femoralis cannula was performed. The artery canalis were rounded from the thigh root of animals to back via subcutaneous tunnel, and were drawn out from head and neck to be fixed. The rats were free to access food for 2 days postoperatively.

The SHR rats were randomly divided into lercanidipine and losartan compound preparation 1 (mass percentage of main ingredient: lercanidipine hydrochloride 50% and losartan potassium 12%), lercanidipine and losartan compound preparation 2 (mass percentage of main ingredient: lercanidipine hydrochloride 30% and losartan potassium 46%), lercanidipine and losartan compound preparation 3 (mass percentage of main ingredient: lercanidipine hydrochloride 0.25% and losartan potassium 58%), 20 rats for each group. Systems were connected to perform awake blood pressure monitoring. Firstly operated for adaptation for 4h. During the monitoring, dosed orally and the dosing time was at 10:00 am. The blood pressure record was started from 1h before dosing and finished at hour 8 after dosing.

### 1.2.2 Detection index

For awake pressure measurement, systolic blood pressure (SBP), diastolic blood pressure (DBP), heartbeat interphase (HP), pulse pressure (PP) and systolic blood pressure variation (SBPV) per pulsation were real time recorded by computer. After 4 h adaptive stable, blood pressure signals per pulsation were started to record. The record time was 9 h (when observing the decreasing blood pressure effects of single dosing yajing capsule acutely, record was from 1h before dosing to hour 8 after dosing, i.e. from 9:00 of the dosing day to 22:00 that day), then the total hemodynamic index was analyzed off-line.

### 1.2.3 Statistical process

SPSS 10.0 statistical software was used to process. The detected data was expressed as mean# standard deviation. Paired t-test was used to compare before and after dosing for each group. One-way ANOVA was used to compare the difference of mean value between each group. And LSD-q test was used to multiple compare the difference of mean value between each group.

### 1.3 Results

The blood pressure variation condition of SHR rats after 3 groups dosing. After orally dosing, the blood pressures for lercanidipine and losartan compound preparation 1 and lercanidipine and losartan compound preparation 3 both changed to some degree comparing with pre-dosing. For lercanidipine and losartan compound preparation 2, the blood pressure changed significantly before and after dosing, and has the statistical significance.

### Example 5: Clinical trial study on lercanidipine and losartan compound preparations not according to the invention

### 1. Material and method

1.1 Selection of cases: patients aged from 18-75 years old with light and moderate diastolic hypertension (supine diastolic blood pressure 95-115 mmHg), excluding severe hypertension (diastolic blood pressure > 115 mmHg), secondary hypertension, heart disease, myocardial infarction, shock, transient ischemic stroke within the past 3 months, sinus bradycardia or sinus tachycardia, hypovolemia, liver and kidney dysfunction and diabetes. After 14-20 days of washing stage, entering 14-30 days of introduction stage, during which single blinded placebos were taken. Blood pressures were measured in a return visit in every 7-10 days. Suitable patients were divided into treatment groups according to random principle.

1.2 Trial method: there were totally 396 patients enrolled, and 388 patients entered the introduction stage and 307 entered the assessment stage. The patients were divided into 4 groups randomly: 1) low dose compound preparation group: a lercanidipine and losartan compound preparation containing 5 mg lercanidipine and 20 mg losartan; 2) high dose single administration group 1: lercanidipine hydrochloride (Recordati Industria Chimica e Farmaceutica S.P.A.), 10 mg; 3) high dose single administration group 2: 100 mg losartan (produced by Hangzhou MSD Co., Ltd.); and 4) positive control group: 10 mg amlodipine (Pfizer Inc.), once daily, continuous administration.

1.3 Observation indicators and method: blood pressures were measured as per the requirements of international standard. A settled physician would follow up once a week and visited from 9:00 to 11:00. Every time heart rate and sitting blood pressure were measured 3 times and the 2 greater values were taken to calculate the mean value.

1.4 Laboratory examinations: electrocardiogram, blood and urine routine, liver and kidney function, blood sugar, blood lipid and blood electrolyte examinations were performed before and after overall patient trial.

1.5 Curative effect assessment: according to the regulations of The National Cardiovascular Epidemiology and Population Prevention report meeting summary in 1979: (1) markedly effective: diastolic blood pressure was decreased by equal to or greater than 10 mmHg, and was decreased to normal (<90 mmHg) or decreased by more than 20 mmHg; (2) effective: diastolic blood pressure was decreased by 10-19 mmHg, or was decreased by less than 10 mmHg, but reached normal; (3) failure: not reached above criteria.

1.6 Data processing and statistical method: the measurement data were calculated as mean ± standard variation. Paired t-test was used to compare the quantitative indicators before and after the 4 treatment patterns. Chi-square test was used to compare the difference before and after administration.

### 2. Results

Analysis of curative effect: (1) change of blood pressure at the end of week 12 of treatment: at the end of week 12 of treatment, blood pressures in all of the 4 groups of patients decreased remarkably, and the differences had statistical significance (p<0.05)(see table 2).

Table 2 shows the changes of average diastolic blood pressure and systolic blood pressure before treatment and at the end of week 12 of treatment in 4 groups of patients, wherein 1) is a lercanidipine and losartan compound preparation containing 5 g lercanidipine and 20 g losartan prepared in Example 1; 2) is 10 mg lercanidipine hydrochloride; 3) is 100 mg lorsartan; and 4) is 10mg amlodipine.

**Table 2 changes of average diastolic blood pressure and systolic blood pressure before treatment and at the end of week 12 of treatment in 4 groups of patients**

| Group | Diastolic blood pressure (mmHg) | | | Systolic blood pressure (mmHg) | | | Effective rate |
|---|---|---|---|---|---|---|---|
| | Before dosing | After dosing | Value changed | Before dosing | After dosing | Value changed | |
| 1)(80 cases) | 106±6 | 85±6 | 21±6 | 156±5 | 130±7 | 25±7 | 85.2% |
| 2)(75 cases) | 104±9 | 88±7 | 13±8 | 152±9 | 139±8 | 19±3 | 70.8% |
| 3)(73 cases) | 100±5 | 81±9 | 11±5 | 152±9 | 138±6 | 15±9 | 75.1% |
| 4)(79 cases) | 104±10 | 89±9 | 15±6 | 153±8 | 135±8 | 11±8 | 74.9% |

| | | | | | | | |
|---|---|---|---|---|---|---|---|
| Note: P<0.05 compared with pre-dosing; P <0.05 compared with amlodipine group. | | | | | | | |

The decline of diastolic blood pressure of the lercanidipine and losartan compound preparation group was larger than that of amlodipine group, and the difference had statistical significance (P<0.05); the systolic blood pressures of the 4 groups also decreased significantly, wherein the decline of systolic blood pressure of the lercanidipine and losartan compound preparation group was remarkably greater than that of amlodipine group, and the difference had statistical significance (P <0.05). The decline of diastolic and systolic blood pressures of the lercanidipine and losartan compound preparation group were significantly greater than those of lercanidipine hydrochloride high dose single administration group and lorsartan high dose single administration group, and the differences had statistical significance (P <0.05).

(2) Comparison of blood pressure decreasing effective rate: the total effective rate for decreasing blood pressure of the lercanidipine and losartan compound preparation group at the end of week 12 of treatment was 85.2%, which was significantly higher than that of the lercanidipine hydrochloride single administration group (70.8%) and the lorsartan single administration group (75.1%) (P <0.05).

There were two cases occurring gastrointestinal tract symptoms and one dizziness in the lercanidipine hydrochloride single administration group. There were four cases occurring gastrointestinal tract symptoms and one dry mouth in the losartan single administration group. All of those were slight and had no effect on continuous dosing. The difference of the incidences of adverse reactions between the two groups were not significant. There was no remarkable adverse reaction for the combined dosing groups, and for the 3 groups of patients treated for 12 weeks, there was no significant difference in laboratory examination results and changes in electrocardiogram.

### 3. Conclusions

The lercanidipine and losartan compound preparation group has significantly effective rate, and the incidence of adverse reactions is significantly decreased, tolerated well by the patients, compared with the high dosage single administration group. Therefore, treatment of mild and moderate hypertension by the lercanidipine and losartan compound preparation could improve curative effect of blood pressure decrease, and it is tolerable and has no remarkable influence on cardiac conductivity and heart rate.

### Example 6: Clinical trial study on lercanidipine and losartan compound preparations not according to the invention

### 1. Material and method

1.1 Selection of cases: patients aged from 18-75 years old with light and moderate diastolic hypertension (supine diastolic blood pressure 95-115 mmHg), excluding severe hypertension (diastolic blood pressure > 115 mmHg), secondary hypertension, heart disease, myocardial infarction, shock, transient ischemic stroke within the past 3 months, sinus bradycardia or sinus tachycardia, hypovolemia, liver and kidney dysfunction and diabetes. After 14-20 days of washing stage, entering 14-30 days of introduction stage, during which single blinded placebos were taken. Blood pressures were measured in a return visit in every 7-10 days. Suitable patients were divided into treatment groups according to random principle.

1.2 Trial method: there were totally 359 patients enrolled, and 305 patients entered the introduction stage and 266 entered the assessment stage. The patients were divided into 4 groups randomly: 1) low dose compound preparation group: a lercanidipine and losartan compound preparation containing 7.5 mg lercanidipine and 20 mg losartan; 2) high dose single administration group 1: lercanidipine hydrochloride (Recordati Industria Chimica e Farmaceutica S.P.A.), 10 mg; 3) high dose single administration group 2: 100 mg losartan (produced by Hangzhou MSD Co., Ltd.).

1.3 Observation indicators and method: blood pressures were measured as per the requirements of international standard. A settled physician would follow up once a week and visited from 9:00 to 11:00. Every time heart rate and sitting blood pressure were measured 3 times and the 2 greater values were taken to calculate the mean value.

1.4 Laboratory examinations: electrocardiogram, blood and urine routine, liver and kidney function, blood sugar, blood lipid and blood electrolyte examinations were performed before and after overall patient trial.

1.5 Curative effect assessment: according to the regulations of The National Cardiovascular Epidemiology and Population Prevention report meeting summary in 1979: (1) markedly effective: diastolic blood pressure was decreased by ≥ 10 mmHg, and was decreased to normal (<90 mmHg) or decreased by more than 20 mmHg; (2) effective: diastolic blood pressure was decreased by 10-19 mmHg, or was decreased by < 10 mmHg, but reached normal; (3) failure: not reached above criteria.

1.6 Data processing and statistical method: the measurement data were calculated as mean ± standard variation. Paired t-test was used to compare the quantitative indicators before and after the 4 treatment patterns. Chi-square test was used to compare the difference before and after administration.

### 2. Results

Analysis of curative effect: (1) change of blood pressure at the end of week 12 of treatment: at the end of week 12 of treatment, blood pressures in all of the 4 groups of patients decreased remarkably, and the differences had statistical significance (P<0.05) (see table 3).

**Table 3 changes of average diastolic blood pressure and systolic blood pressure before treatment and at the end of week 12 of treatment in 3 groups of patients**

| Group | Diastolic blood pressure (mmHg | | | Systolic blood pressure (mmHg) | | | Effective rate |
|---|---|---|---|---|---|---|---|
| | Before dosing | After dosing | Value changed | Before dosing | After dosing | Value changed | |
| 1)(89 cases) | 105±9 | 86±8 | 23±8 | 158±8 | 133±9 | 28±9 | 87.4% |
| 2)(90 cases) | 106±8 | 89±7 | 16±6 | 156±8 | 136±7 | 19±7 | 71.6% |
| 3)(87 cases) | 102±6 | 83±8 | 14±5 | 152±7 | 139±5 | 17±5 | 77.3% |

| | | | | | | | |
|---|---|---|---|---|---|---|---|
| Note: P<0.05 compared with pre-dosing. | | | | | | | |

Wherein, 1) is a lercanidipine and losartan compound preparation containing 7.5 g lercanidipine and 20 g losartan; 2) is 10 mg lercanidipine hydrochloride; and 3) is 100 mg lorsartan.

The decline of diastolic blood pressure of the lercanidipine and losartan compound preparation group was larger than that of amlodipine group, and the difference had statistical significance (P<0.05); the systolic blood pressures of the 3 groups also decreased significantly, wherein the decline of systolic blood pressure of the lercanidipine and losartan compound preparation group was remarkably greater than that of amlodipine group, and the difference had statistical significance (P <0.05). The decline of diastolic and systolic blood pressures of the lercanidipine and losartan compound preparation group were significantly greater than those of lercanidipine hydrochloride high dose single administration group and lorsartan high dose single administration group, and the differences had statistical significance (P <0.05).

(2) Comparison of blood pressure decreasing effective rate: the total effective rate for decreasing blood pressure of the lercanidipine and losartan compound preparation group at the end of week 12 of treatment was 87.4%, which was significantly higher than that of the lercanidipine hydrochloride single administration group (71.6%) and the lorsartan single administration group (77.3%) (P <0.05).

There was one case occurring gastrointestinal tract symptoms and one dizziness in the lercanidipine hydrochloride single administration group. There were two cases occurring gastrointestinal tract symptoms and one dry mouth in the losartan single administration group. All of those were slight and had no effect on continuous dosing. The difference of the incidences of adverse reactions between the two groups were not significant. There was no remarkable adverse reaction for the combined dosing groups, and for the 3 groups of patients treated for 12 weeks, there was no significant difference in laboratory examination results and changes in electrocardiogram.

### 3. Conclusions

The lercanidipine and losartan compound preparation group has significantly effective rate, and the incidence of adverse reactions is significantly decreased, tolerated well by the patients, compared with the high dosage single administration group. Therefore, treatment of mild and moderate hypertension by the lercanidipine and losartan compound preparation could improve curative effect of blood pressure decrease, and it is tolerable and has no remarkable influence on cardiac conductivity and heart rate.

## Claims

1. A lercanidipine and losartan compound preparation, **characterized in that** lercanidipine hydrochloride accounts for 0.5-40% and losartan potassium accounts for 6.25-50%, based on mass percentage of the compound preparation, and the mass ratio of lercanidipine hydrochloride to losartan potassium as active pharmaceutical ingredients in the compound preparation is 30:46.

2. The compound preparation according to claim 1, **characterized in that** excipients used are 20-40% lactose monohydrate, 10-40% microcrystalline cellulose, 10-20% A-type sodium starch glycolate, 2-8% povidone K30, 0.5-2.5% magnesium stearate, 5-15% pregelatinized starch and 1-10% colloidal silicon dioxide.

## Patentansprüche

1. Lercanidipin- und Losartan-Kombinationspräparat, **dadurch gekennzeichnet, dass** Lercanidipin-Hydrochlorid 0,5-40% ausmacht und Losartan-Kalium 6,25-50% ausmacht, bezogen auf die Masse des Kombinationspräparats, und das Massenverhältnis von Lercanidipin-Hydrochlorid zu Losartan-Kalium als pharmazeutische Wirkstoffe in dem Kombinationspräparat 30:46 beträgt.

2. Kombinationspräparat gemäß Anspruch 1, **dadurch gekennzeichnet, dass** es sich bei den verwendeten Arzneimittelhilfsstoffen um 20-40% Lactosemonohydrat, 10-40% mikrokristalline Cellulose, 10-20% Natriumstärkeglycolat Typ A, 2-8% Povidon K30, 0,5-2,5% Magnesiumstearat, 5-15% vorgelatinisierte Stärke und 1-10% kolloidales Siliciumdioxid handelt.

## Revendications

1. Préparation composée de lercanidipine et de losartan, **caractérisée en ce que** le chlorhydrate de lercanidipine représente de 0,5 à 40 % et le potassium de losartan représente de 6,25 à 50 %, par rapport au pourcentage en masse de la préparation composée, et le rapport en masse entre le chlorhydrate de lercanidipine et le potassium de losartan en tant qu'ingrédients pharmaceutiques dans la préparation composée est de 30:46.

2. Préparation composée selon la revendication 1, **caractérisée en ce que** les excipients utilisés sont le monohydrate de lactose à 20 à 40 %, la cellulose microcristalline à 10 à 40 %, le glycolate d'amidon sodique de type A à 10 à 20 %, la povidone K30 à 2 à 8 %, le stéarate de magnésium à 0,5 à 2,5 %, l'amidon prégélatinisé à 5 à 15 % et le dioxyde de silicium colloïdal à 1 à 10 %.
